# EUROPEAN PATENT APPLICATION

(11) **EP 3 954 349 A1**
(43) Date of publication of application: **16.02.2022**
(21) Application number: 20190595.7
(22) Date of filing: 11.08.2020
(51) Int. Cl.: A61F 13/15, A61F 13/47

(54) **REUSABLE SANITARY PAD**

(71) Applicant: Sanitary Owl Ltd (T/A DAME), London SE1 0NZ (GB)
(72) Inventor: Mills, Alexander, London, London SE1 0NZ (GB); Williamson, Anna, London, London SE1 0NZ (GB)
(74) Representative: Orrin, Alexandra Louise Helena

(57) **Abstract**

There is provided a reusable sanitary pad, comprising an upper fabric layer and a lower absorbent layer fixedly attached to the upper fabric layer. The lower absorbent layer is configured to absorb and/or store fluid. The upper fabric layer comprises: a perimeter component comprising a perimeter material, having an aperture; and an insert component comprising an insert material that is different to the perimeter material, and that is configured to conduct fluid away from an upper surface of the upper fabric layer towards the absorbent layer. The insert component is disposed across the aperture and fixedly attached to the perimeter component.

## Description

### FIELD OF THE INVENTION

The present invention relates to a reusable pad, in particular but not limited to a reusable sanitary pad.

### BACKGROUND OF THE INVENTION

Sanitary pads, also known as sanitary towels, are products that are known to collect and retain bodily fluid, such as menstrual blood or urine. These known pads are designed so that when a pad has reached its fluid retention limit, it is disposed of by a user. The main design priorities for these known disposable pads have been reliability of fluid retention, hygiene and comfort of the user, and production cost. The materials used in these known pads are often chosen with these design priorities in mind. Common materials used in known disposable pads are synthetic polymer-based materials, such as poly(propylene) or poly(ethylene). In some cases, a majority of the pad can comprise plastic, in particular plastic that is not biodegradable. These known disposable pads have associated environmental issues, as they are typically not designed to degrade after use.

Reusable pads have been developed in an attempt to solve the environmental issues caused by disposable pads. One known reusable pad has an upper fluid conducting fabric layer, a middle absorbent layer, and a lower waterproof layer. This known product is designed to be washed and re-used, and so avoids or limits the known environmental problems associated with disposable pads. However, the inventors have identified that this known product can have problems associated with reliability of fluid retention and hygiene. The inventors have also identified that this known product has problems associated with comfort of the user, as it is bulky. This known product also has an upper fabric which can crease and wrinkle, so can cause discomfort.

There therefore exists a need for improvements in sanitary pads.

### SUMMARY OF THE INVENTION

A first aspect of the invention provides a reusable sanitary pad, comprising an upper fabric layer and a lower absorbent layer fixedly attached to the upper fabric layer; the lower absorbent layer being configured to absorb and/or store fluid;
wherein the upper fabric layer comprises:
a perimeter component comprising a perimeter material, having an aperture;
an insert component comprising an insert material that is different to the perimeter material, and that is configured to conduct fluid away from an upper surface of the upper fabric layer towards the absorbent layer, the insert component being disposed across the aperture and fixedly attached to the perimeter component.

The inventors have identified that by providing an insert component across an aperture of a perimeter component, where the insert component comprises an insert material that is different to the perimeter material, and is configured to conduct fluid away from an upper surface of the upper fabric layer, a particularly effective sanitary pad is provided. The combination of the perimeter component having an aperture and an insert material across the aperture provides a pad in which the fluid conducting material conducts fluid away from the user at a particularly suitable rate. By controlling the rate at which fluid is conducted away from the body, and by controlling the location at which fluid is first conducted away from the body, user comfort, reliability of fluid retention and hygiene properties of the pad are significantly improved. The user is therefore provided with a reusable pad which avoids the environmental concerns of known disposable pads, but does not suffer from the typical problems of reusable pads, i.e. quality of the product and comfort of the user. The user is also provided with a pad that is less bulky and is more flexible than known reusable pads, so is more comfortable for the user.

The perimeter component and the insert component may each have an upper surface, which together define an upper surface of the pad. This has the advantage of providing further control over the rate of fluid distribution through the reusable pad.

The insert material may be configured to conduct fluid away from an upper surface of the upper fabric layer by means of a wicking mechanism. This has the advantage of providing a particularly effective fluid flow pathway through the pad, and particularly effective control of fluid distribution through the reusable pad. By providing an insert material configured to wick fluid away from the user, the user is also provided with a soft-to-touch material surface, which improves user comfort.

The insert component may be wholly surrounded in plan view by the perimeter component. This benefits from the advantages provided above. This also provides improved user comfort, while benefiting from providing a product that is robust and easy and cost-effective to manufacture.

The insert component and the perimeter component may be fixedly attached to one another so as to substantially define a plane.

The pad may further comprise a lower fabric layer. The absorbent layer may be disposed between the upper fabric layer and the lower fabric layer. This has the advantage of improving user comfort, and has aesthetic advantages in terms of the simplicity of the outer casing of the product. This also provides a more robust reusable pad which may be washed and reused over a longer time.

The pad may further comprise a protective layer disposed to restrict and/or prevent fluid. The protective layer may be disposed beneath the absorbent layer. This has the advantage of providing a reusable pad having particularly effective fluid retention properties. This also has the advantage of providing control over the fluid distribution path through the reusable pad.

The insert component may be substantially planar and may define an elongate shape. The insert component may substantially define a rounded, substantially oval, shape. This has the advantage of allowing fluid to be conducted quickly away from the body at an initial contact point of the reusable pad, which improves user comfort.

The insert component and/or the aperture may be disposed in a substantially central position relative to the perimeter component. This has the advantage noted above for the shape of the insert component, and additionally has the advantage of providing a structurally robust arrangement, which is particularly suitable for washing and re-use.

The insert component may have an outer edge. The aperture may be substantially aligned with the outer edge of the insert component. This alignment may be such that the insert component and the aperture define substantially the same shape in plan view. The insert component may be configured to at least partly overlap with the perimeter component. This has the advantage of providing a lightweight arrangement that is easy to manufacture, and structurally suitable for washing and re-use.

The or each fabric layer may be a woven fabric layer. The insert component may be fixedly attached to the perimeter component by means of stitching. This has the advantage of providing a structurally robust arrangement, which is particularly suitable for washing and re-use.

The pad may further comprise an attachment means for attachment of the reusable sanitary pad to a garment.

A second aspect of the invention provides a method of manufacturing a reusable sanitary pad, comprising:
providing a lower absorbent layer; a perimeter component comprising a perimeter material, having an aperture; an insert component comprising an insert material that is different to the perimeter material,
fixedly attaching the perimeter component to the insert component so as to form an upper fabric layer, such that the insert component is disposed across the aperture; and
fixedly attaching the upper fabric layer to the lower absorbent layer.

This aspect provides a pad having at least the advantages noted above in relation to the first aspect.

The method may further comprise providing a lower fabric layer, and fixedly attaching the lower fabric layer to the upper fabric layer.

Fixedly attaching the perimeter component to the insert component, and fixedly attaching the absorbent layer to the upper fabric layer, may be performed simultaneously. This has the advantage of providing a simple manufacturing method, which results in a robust attachment between the perimeter component, the absorbent layer and the upper fabric layer.

### BRIEF DESCRIPTION OF THE DRAWINGS

Embodiments of the invention will now be described with reference to the accompanying drawings, in which:
Figure **1** shows an upper fabric layer of a reusable sanitary pad;
Figure **2** shows a lower fabric layer of a reusable sanitary pad;
Figure **3** shows an expanded view of component parts of a reusable sanitary pad; and
Figure **4** shows an enlarged view of the upper fabric layer of Figure 3.

### DETAILED DESCRIPTION OF EMBODIMENT(S)

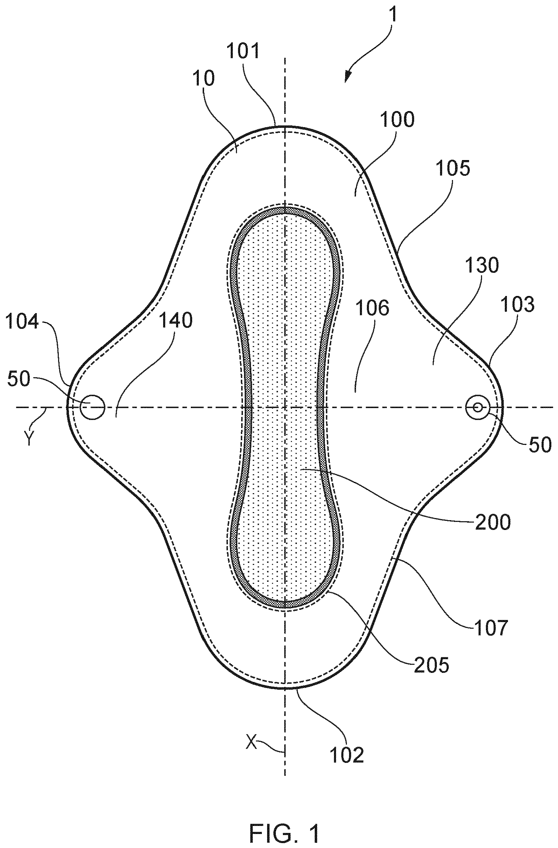

With reference to Figure 1, the reusable sanitary pad 1 comprises an upper fabric layer 10. The upper fabric layer 10 comprises a perimeter component 100 and an insert component 200.

The perimeter component 100 comprises a perimeter material. The perimeter material may be substantially or moderately hydrophilic. The perimeter component 100 may comprise or consist of a cellulose fibre and/or cotton, or a cellulose fibre and cotton blend. However, the skilled person will appreciate that various appropriate materials for the perimeter component 100 could be used. The cellulose fibre may be Lyocell (RTM). The perimeter component 100 may comprise an antimicrobial, i.e. an antibacterial, additive. The perimeter component 100 may be a dark colour, or may have been dyed to a dark colour.

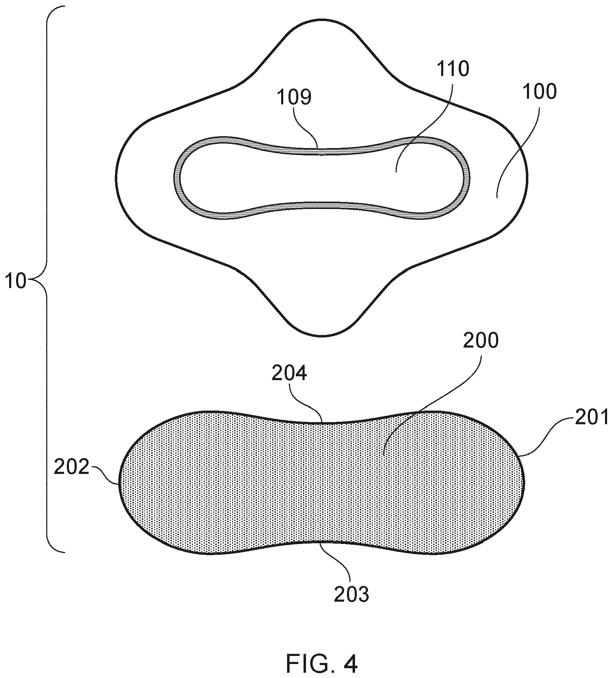

The perimeter component 100 has an aperture 110, for example as shown in figure 4, which may be an opening, such as an internal opening. The perimeter component 100 is substantially planar and has an outer edge 105. The perimeter component 100 has a first end 101, a second end 102, a first side 103 and a second side 104. The perimeter component 100 may be configured to have a curved outer edge 105 at its first and/or its second end 101, 102. The outer edge 105 of the perimeter component 100 may be convex at its first and/or its second end 101, 102. The pad 1 may have a longitudinal axis X, which may extend from the first end 101 to the second end 102. The perimeter component 100 may be substantially symmetrical about its longitudinal axis X.

The perimeter component 100 may be configured to have a curved outer edge 105 at its first and/or its second side 103, 104. The outer edge 105 of the perimeter component 100 may be convex at its first and/or its second side 103, 104. The first side 103 and/or the second side 104 of the perimeter component 100 may be foldable relative to a body 106 of the outer perimeter 100. The body 106 may be any part of the pad 1 which is configured to be located above a garment in use. There may be provided a first wing 130 and a second wing 140. The first wing 130 may be provided on the first side 103 and the second wing 140 may be provided on the second side 104. The first and/or second wing 130, 140 may be foldable relative to the body 106 of the outer perimeter component 100. The first and/or second wing 130, 140 may be dimensioned so as to overlap with one another when folded about the body 106. The pad 1 may have a lateral axis Y, which may extend from the first side 103 to the second side 104. The perimeter component 100 may be substantially symmetrical about the lateral axis Y. The outer edge 105 of the perimeter component 100 may be configured having at least one concave portion. There may be provided at least one concave portion between: the first end 101 and the first side 103, the first side 103 and the second end 102, the second end 102 and the second side 104, and/or the second side 104 and the first end 101.

There may be provided an attachment means 50 for attaching the pad 1 to a garment. The attachment means 50 may be provided on the first and/or second wing 130, 140. The attachment means 50 may comprise an attachment component including but not limited to: a snap fit connection; an interference connection; a button and button hole and/or a hook and loop attachment, such as Velcro (RTM). The attachment means 50 may comprise a popper, which may comprise a stud and socket. The first wing 130 may be attachable to the second wing 140 by means of the attachment means 50. The first and/or second wing 130, 140 and/or the attachment means may be configured such that when a garment is provided between the body 106 of the pad 1 and the first and/or second wing 130, 140, and when the first and second wing 130, 140 are attached or secured to one another by means of an attachment means 50, the pad may be secured in place, relative to the garment.

As shown in Figure 1, the perimeter component 100 may have an upper surface which may define an upper surface of the pad 1. The perimeter component 100 may comprise an inner edge 109, as best seen in figure 4, which defines the aperture 110. The inner edge 109 may have a surface texture and/or be visually identifiable as having been cut to shape and/or size. The inner edge 109 may have a seam and/or stitching.

The aperture 110 of the perimeter component 100 may be disposed in a substantially central position relative of the perimeter component 100, although a skilled person will appreciate that other positions are possible. The aperture 110 may be disposed in a position within the perimeter component 100 such that when the pad 1 is worn, the aperture 110 is aligned with the most likely area or place for initial contact with fluid such as menstrual fluid or urine, or other bodily fluid.

The aperture 110 may extend partly from the perimeter first end 101 towards the perimeter second end 102. The aperture 110 may define a substantially elongate shape, such as but not limited to an oval, a rectangle, or a rounded rectangle. The aperture 110 may have a tapered middle section. The aperture 110 may be a substantially rounded figure of eight shape, i.e. be substantially rounded lemniscate. The aperture 110 may define a width in the Y direction, and may have a smaller width on or proximate the Y axis than away from the Y axis. This may provide a shape having at least one enlarged end, which may be proximate the first end 101 and/or second end 102. The aperture may comprise at least one, preferably two concave portions and at least one, preferably two convex portions. The concave portion or portions may extend in the X direction and/or be intersected by the Y axis. The convex portion or portions may extend in the Y direction and/or be intersected by the X axis. The aperture 110 may be curved such that it is convex at or proximate the first end 101, convex at or proximate the second end 102, concave at or proximate the first side 103, and/or concave at or proximate the second side 104.

The aperture 110 may be wholly surrounded in plan view by the perimeter component 100. The skilled person will appreciate that "plan view" refers to a view from above, for example as shown in figure 1. The aperture 110 may be an internal aperture, for example as shown in figure 1. The aperture 110 may be disposed wholly within the body 106 of the perimeter component. The aperture 110 may not be disposed in the first wing 130 and/or the second wing 140.

The insert component 200 comprises an insert material. The insert material is different to the perimeter material. The insert material is configured to conduct fluid away from an upper surface of the upper fabric layer 10. The insert material may be configured to conduct fluid away from an upper surface of the upper fabric layer 10 by means of a wicking mechanism. The insert material may comprise or consist of poly(amide), poly(ester) and/or a poly-ether - poly-urea copolymer, such as but not limited to Lycra (RTM). The insert material may comprise hydrophilic and/or hydrophobic material, which may comprise or consist of hydrophilic and/or hydrophobic fibres. The insert material may define a plurality of holes or capillary channels to enable fluid to be wicked. The insert material may comprise one or more hydrophilic fibres to enable fluid to be wicked. The insert material may be configured to not wick fluid in all directions, and/or to wick fluid only in substantially one direction. The insert material may be configured to wick fluid away from an upper surface of the pad 1, towards an absorbent material or layer 30. The insert material may be configured to prevent or restrict fluid from passing from the absorbent material or layer 30 towards the upper surface of the pad 1. The insert component 200 may comprise an antimicrobial, i.e. an antibacterial, additive. The insert component 200 may be a dark colour, or may have been dyed to a dark colour.

The insert component 200 may be substantially planar. The insert component 200 may have a corresponding shape to the shape of the aperture 110 described above.

As shown in Figure 1, the insert component 200 is disposed across the aperture 110, and is fixedly attached to the perimeter component 100. By being arranged in this way, the insert component 200 and the perimeter component 100 may each have an upper surface which together define an upper surface of the pad 1. The insert component 200 may have an outer edge 205. The outer edge 205 may be substantially aligned with the aperture 110. The insert component 200 and the aperture 110 define substantially the same shape in plan view. The insert component 200 may be larger than the aperture 110. The insert component 200 may be a scaled-up shape of the aperture 110. The insert component 200 may comprise a first end 201, a second end 202, a first side 203 and/or a second side 204, as best seen in Figure 4. The first end 201 of the insert component 200 may be arranged at or proximate the first end 101 of the perimeter component 100. The second end 202 of the insert component 200 may be arranged at or proximate the second end 102 of the perimeter component 100. The third side 203 of the insert component 200 may be arranged at or proximate the first side 103 of the perimeter component 100. The fourth end 204 of the insert component 200 may be arranged at or proximate the fourth end 104 of the perimeter component 100.

As shown in Figure 1, the insert component 200 may be wholly surrounded in plan view by the perimeter component 100. The insert component 200 may be disposed in a substantially central position relative to the perimeter component 100. The insert component 200 may overlap at least partly with the perimeter component 100. Specifically, the outer edge 205 of the insert component 200 may overlap at least partly with the inner edge 109 of the perimeter component 110. This overlap may be such that at least part, or all of the outer edge 205 of the insert component 200 overlaps with the perimeter component 100. The overlap may be such that the insert component 200 is below the perimeter component 100. Equally, the overlap may be such that the insert component 200 is above the perimeter component 100. The overlap may have a radial dimension which is substantially uniform around the entire insert component edge 205.

The insert component 200 and the perimeter component 100 may be fixedly attached to one another so as to substantially define a plane. The insert component 200 may be fixedly attached to the perimeter component 100 by means of sewing or stitching, such as by an overlock stitch. As the skilled person will appreciate, any appropriate attaching means and/or stitching or sewing type may be used.

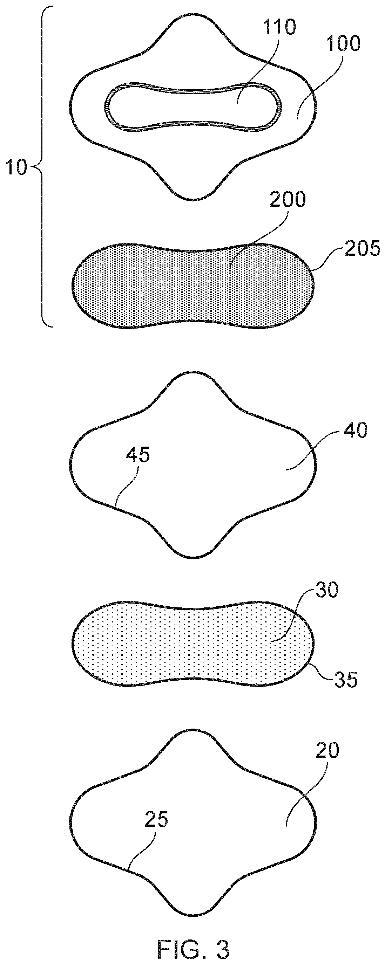

With reference to Figure 3, the reusable sanitary pad 1 further comprises a lower absorbent layer 30, which may be an absorbent fabric layer. The absorbent layer 30 is configured to absorb and/or store fluid. The absorbent layer 30 is fixedly attached to the upper fabric layer 10. The absorbent layer 30 may be fixedly attached to the upper fabric layer 10 directly or indirectly. The absorbent layer 30 may be fixedly attached to an intermediate layer 40, which may be attached to the upper fabric layer 10. The absorbent layer 30 is arranged relative to the upper fabric layer 10 such that the insert component 200 can conduct fluid away from an upper surface of the upper fabric layer 10 towards the absorbent layer 30. The absorbent layer 30 may be substantially the same size and/or shape as the insert component 200. The absorbent layer 30 may have an outer edge 35. The absorbent layer 30 may comprise a different material to the insert component 200 and/or to the perimeter component 100. The absorbent layer 30 may comprise and/or consist of one or more of: a microfibre fabric; cotton, such as organic cotton; a cotton or organic cotton blend; bamboo, such as bamboo viscose or bamboo rayon; polyester; bamboo terry fabric such as bamboo terry jersey.

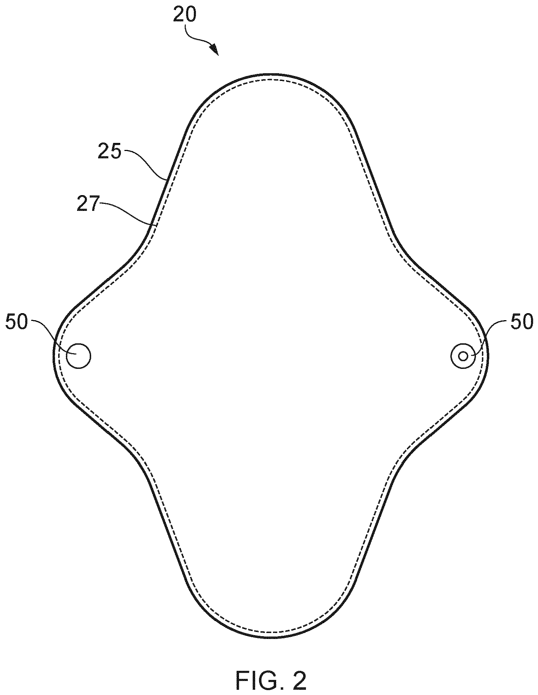

The pad 1 may further comprise a lower fabric layer 20. The lower fabric layer 20 may be as shown in Figure 2, in which a rear view of the pad 1 is shown. The lower fabric layer 20 may comprise or consist of the same material as the perimeter component 100. The lower fabric layer 20 may have an outer edge 25 which may be substantially the same shape and and/or size as the outer edge 105 defined by the perimeter component 100. The lower fabric layer 20 may be attached to the upper fabric layer 10. This attachment may be by means of stitching, which may comprise internal stitching and/or a top stitch 27.

The lower fabric layer 20 may comprise a gripping means, such as a surface roughness or texture. For example, the lower fabric layer may comprise a pattern, which may be defined by a polymer such as, but not limited to, silicone. The lower fabric layer 20 may be arranged such that the absorbent layer 30 is disposed between the upper fabric layer 10 and the lower fabric layer 20, for example as depicted in Figure 3. The pad 1 may further comprise a protective layer disposed to restrict and/or prevent fluid. This protective layer may be a coating, which may be disposed beneath the absorbent layer 30. The protective layer may be disposed between the absorbent layer 30 and the lower fabric layer 20.

There may be provided an intermediate layer 40, which may be the same shape, size, and/or material as the lower fabric layer 20. The intermediate layer 40 may have an outer edge 45. The intermediate layer 40 may be arranged between the upper layer 10 and the absorbent material 30.

In an assembled state, the perimeter component 100, the insert component 200, the intermediate layer 40 and/ or the absorbent layer 30 may be fixedly attached to one another. The lower fabric layer 20 may be fixedly attached to the perimeter component 100. In an assembled state, the outer edge 205 of the insert may be substantially aligned with the outer edge 35 of the absorbent layer 30. Equally, in an assembled state, the outer edge 205 of the insert may not be substantially aligned with the outer edge 35 of the absorbent layer 30. At least two, or all, of the following may be at least partly or wholly aligned: the outer edge 105 of the perimeter component 100, the outer edge 45 of the intermediate component 40, the outer edge 25 of the lower component 20.

The pad 1 may be configured to be worn such that the first end 101 is the front end, and the second end is the back end 102. The pad 1 may be configured such that the first and second ends 101, 102 and body 106 of the pad 1 can be aligned with underwear.

With reference to figure 3, a pad 1 is be provided using a method comprising:
providing a lower absorbent layer 30; a perimeter component 100 comprising a perimeter material, having an aperture 110; an insert component 200 comprising an insert material that is different to the perimeter material,
fixedly attaching the perimeter component 100 to the insert component 200 so as to form an upper fabric layer 10, such that the insert component 200 is disposed across the aperture 110; and
fixedly attaching the upper fabric layer 10 to the lower absorbent layer 30.

The method may further comprise providing a lower fabric layer 20, and fixedly attaching the lower fabric layer 20 to the upper fabric layer 10.

Fixedly attaching the perimeter component 100 to the insert component 200, and fixedly attaching the absorbent layer 30 to the upper fabric layer 10, may be performed simultaneously.

The method may comprise any or all of the following steps, performed in the order below and/or any appropriate order:
a) providing one or more of: a lower absorbent layer 30; a perimeter component 100 comprising a perimeter material, having an aperture 110; an insert component 200 comprising an insert material that is different to the perimeter material; a lower fabric layer 20, and an intermediate layer 40.
b) adding a stitch, such as an overlocking stitch to the perimeter component 100. This may be along or over an inner edge 109 of the perimeter component 110.
c) aligning one or more or all of: the insert component 200; the intermediate layer 40; the perimeter component 100; the absorbent layer 30.
d) attaching the components aligned in step (c) using a stitch, such as a lock stitch. This may be done over the stitch of step (b). This may be done along an outer edge 205 of the insert component 200.
e) attaching the attached parts of step (d) to the lower fabric layer 20. This may be done by stitching 107, 27 at least partly along an outer edge 105, 25 of the perimeter component 100 and the lower fabric layer 20. This may be carried out such that at least part of the outer edge 105, 25 does not have stitching 107, 27, so that the layers can be turned inside out through a hold defined by the stitching 107, 27.
f) adding a stitch, such as a top stitch, around at least part, preferably the entire, outer edge 25, 105 of the pad 1.
g) attaching at least one attachment means or component 50, such as a popper. This attachment may be done to one or more wings 130, 140 of the perimeter component 100.

Step (c) above may involve aligning an outer edge 205 of the insert component 200 to an outer edge 35 of the absorbent layer 35. Step (c) above may involve aligning an outer edge 45 of the intermediate layer 40 with an outer edge 105 of the perimeter component 100.

Although stitching or sewing has been provided as a particularly advantageous attachment method, the skilled person will appreciate that any appropriate attachment mechanism could be used.

Although the invention has been described above with reference to one or more preferred embodiments and method steps, it will be appreciated that various changes or modifications may be made without departing from the scope of the invention as defined in the appended claims.

Although an embodiment involving an aperture 110 in the perimeter component 100 has been described, the skilled person would appreciate that a fluid conducting material could be provided within the aperture 110 of the perimeter component 100. Equally, the perimeter component 100 may be provided with perimeter material or other material within or across the aperture, and the insert component 200 may be provided on top of the perimeter component 100.

Although an embodiment having wings 130, 140 is described, the skilled person will appreciate that other embodiments without one or more wings 130, 140 are envisaged.

Where the word 'or' appears, this is to be construed to mean 'and/or' such that items referred to are not necessarily mutually exclusive and may be used in any appropriate combination.

As a skilled person will appreciate, the terms "upper", "lower" and "side" are merely guiding terms to show possible, and in some cases preferred, locations in use. These terms should not be read as being limiting or restrictive, as the orientation of the product may change during manufacture or storage.

Reference numerals used in the claims should be construed as a guide to a possible embodiment or embodiments only, and not be construed as limiting on the scope of the claims.

## Claims

1. A reusable sanitary or incontinence pad (1), comprising an upper fabric layer (10) and a lower absorbent layer (30) fixedly attached to the upper fabric layer (10); the lower absorbent layer (30) being configured to absorb and/or store fluid;
wherein the upper fabric layer (10) comprises:
a perimeter component (100) comprising a perimeter material, having an aperture (110);
an insert component (200) comprising an insert material that is different to the perimeter material, and that is configured to conduct fluid away from an upper surface of the upper fabric layer (10) towards the absorbent layer (30), the insert component (200) being disposed across the aperture (110) and fixedly attached to the perimeter component (100).

2. The pad (1) of claim 1, wherein the perimeter component (100) and the insert component (200) each have an upper surface, which together define an upper surface of the pad (1).

3. The pad (1) of claim 1 or claim 2, wherein the insert material is configured to conduct fluid away from an upper surface of the upper fabric layer (10) by means of a wicking mechanism.

4. The pad (1) of any of the preceding claims, wherein the insert component (200) is wholly surrounded in plan view by the perimeter component (100).

5. The pad (1) of any of the preceding claims, wherein the insert component (200) and the perimeter component (100) are fixedly attached to one another so as to substantially define a plane.

6. The pad (1) of any of the preceding claims, further comprising a lower fabric layer (20), wherein the absorbent layer (30) is disposed between the upper fabric layer (10) and the lower fabric layer (20).

7. The pad (1) of any of the preceding claims, further comprising a protective layer disposed to restrict and/or prevent fluid, disposed beneath the absorbent layer (30).

8. The pad (1) of any of the preceding claims, wherein the insert component (200) is substantially planar and defines an elongate shape, optionally a rounded substantially oval shape.

9. The pad (1) of any of the preceding claims, wherein the insert component (200) and/or the aperture (110) are disposed in a substantially central position relative to the perimeter component (100).

10. The pad (1) of any of the preceding claims, wherein the insert component (200) has an outer edge (205), and the aperture (110) is substantially aligned with the outer edge (205), such that the insert component (200) and the aperture (110) define substantially the same shape in plan view,
wherein the insert component (200) is optionally configured to at least partly overlap with the perimeter component (100).

11. The pad (1) of any of the preceding claims, wherein the or each fabric layer (10, 20) is a woven fabric layer, wherein the insert component (200) is optionally fixedly attached to the perimeter component (100) by means of stitching.

12. The pad (1) of any of the preceding claims, further comprising an attachment means (50) for attachment of the reusable sanitary pad (1) to a garment.

13. A method of manufacturing a reusable sanitary pad (1), comprising:
providing a lower absorbent layer (30); a perimeter component (100) comprising a perimeter material, having an aperture (110); an insert component (200) comprising an insert material that is different to the perimeter material,
fixedly attaching the perimeter component (100) to the insert component (200) so as to form an upper fabric layer (10), such that the insert component (200) is disposed across the aperture (110); and
fixedly attaching the upper fabric layer (10) to the lower absorbent layer (30).

14. The method of claim 13, further comprising providing a lower fabric layer (20), and fixedly attaching the lower fabric layer (20) to the upper fabric layer (10).

15. The method of claim 13 or 14, wherein fixedly attaching the perimeter component (100) to the insert component (200), and fixedly attaching the absorbent layer (30) to the upper fabric layer (10), are performed simultaneously.
